# EUROPEAN PATENT APPLICATION

(11) **EP 1 886 622 A1**
(43) Date of publication of application: **13.02.2008**
(21) Application number: 06729151.8
(22) Date of filing: 15.03.2006
(51) Int. Cl.: A61B 1/12, A61L 2/26

(54) **ENDOSCOPE STERILIZATION APPRAISAL APPARATUS**

(30) Priority: 30.05.2005 JP 2005158164
(71) Applicant: OLYMPUS MEDICAL SYSTEMS CORP., Shibuya-ku Tokyo 151-0072 (JP)
(72) Inventor: OGAWA, Akihisa, c/o Olympus Medical Systems Corp., Tokyo 151-0072 (JP); HATORI, Tsuruo, c/o Olympus Medical Systems Corp., Tokyo 151-0072 (JP); TSUJIYA, Hideki, c/o Olympus Medical Systems Corp., Tokyo 151-0072 (JP); WATANABE, Hitoshi, c/o Olympus Medical Systems Corp., Tokyo 151-0072 (JP)
(74) Representative: von Hellfeld, Axel
(86) International application number: PCT/JP2006/305130
(87) International publication number: WO 2006/129406

(57) **Abstract**

An endoscope sterilization evaluation device is provided with an elongated hollow body having a hollow portion opened at opposite ends, the elongated hollow body simulating an endoscope having a tubular passage, an exterior member having a first housing portion in which a biological indicator for performing sterilization evaluation on the hollow body is housed, and a second housing portion in which a culture solution for cultivating germs attached to the biological indicator is housed, the exterior member also having at a predetermined position a communication passage for communication between the first housing portion and the outside, and a mount device for holding the communication passage in a state of communicating with the hollow portion, and mounting the exterior member to the hollow body.

## Description

### Technical Field

The present invention relates to a sterilization evaluation device for checking the sterilization effect of sterilization processing such as high-temperature high-pressure steam sterilization or ethylene oxide gas sterilization on a medical instrument requiring sterilization processing when the sterilization processing is performed by replacing air in a chamber constituting a sterilization unit with a different gas and, more particularly, to an endoscope sterilization evaluation device suitable for checking the sterilization effect on a tubular passage provided in an endoscope.

### Background Art

Apparatuses for performing sterilization such as high-temperature high-pressure steam sterilization (hereinafter referred to as autoclave sterilization) or ethylene oxide gas sterilization (hereinafter referred to as EOG sterilization) by replacing air in a chamber constituting a sterilization unit with a different gas is known as means for sterilization of medical instruments. For example, an autoclave sterilization apparatus fills a chamber with high-temperature high-pressure steam to expose a medical instrument to the steam for a predetermined time, thereby achieving a sterilized condition of the medical instrument. In the case of EOG sterilization, a chamber is filled with ethylene oxide gas and a medical instrument is exposed to the gas for a predetermined time, thereby achieving a sterilized condition of the medical instrument. Sterilization processing on a medical instrument is ordinarily accompanied with an operation for checking after sterilization processing whether or not the medical instrument is reliably sterilized, i.e., whether or not a sufficiently high sterilization effect is obtained.

Conventionally, a chemical indicator for chemically checking the sterilization effect or a biological indicator for biologically checking the sterilization effect is used in an operation to check the sterilization effect.

For example, a chemical indicator having a chemical agent applied to a portion of a paper sheet and dried is known. The chemical indicator is designed so that the chemical agent portion changes in color when exposed to a predetermined sterilized condition. On the other hand, for example, a biological indicator having a predetermined number of germs (referred to as index germs) impregnated into a portion of a paper sheet and dried is known.

In a case where the sterilization effect is checked by means of a chemical indicator or a biological indicator, the indicators are placed in a chamber of a sterilization unit together with a medical instrument to be sterilized. In ordinary cases, a medical instrument is put in a special bag (mentioned as a sterilization pack) or in a special box (mentioned as a sterilization container) when sterilized. When the effect of sterilization of the medical instrument put in the sterilization pack or the sterilization container is to be checked, a chemical indicator or a biological indicator is put in the sterilization pack or the sterilization container together with the medical instrument.

In a chemical indicator, a change in color of a chemical agent applied to the indicator is checked after sterilization of a medical instrument. In this way, a determination can be made as to whether or not the medical instrument has been exposed to a predetermined sterilization condition. That is, the chemical indicator is capable of obtaining a sterilization result immediately after sterilization.

In contrast, obtaining a result with a biochemical indicator requires checking, for example, whether or not all index germs attached to paper have died. Also, checking as to whether or not all index germs attached to paper have died requires immersing the biological indicator in a culture solution and cultivating index germs. If no growth of index germs is recognized after cultivation, it can be recognized that all the index germs have died, and that sterilization has been achieved. Thus, a biochemical indicator is incapable of obtaining a result immediately after sterilization of a medical instrument because of the execution of cultivation operation. Also, when germs are cultivated by immersing the biological indicator in a culture solution, it is necessary to perform the operation in a germ-free environment in order to prevent other germs from falling into or attaching to the indicator or the culture solution. That is, checking the sterilization effect requires a special environment in an aseptic condition and a skilled operator.

In Japanese Patent Laid-Open No. 7-148232 is proposed a biotic indication device with which even an unskilled person can check sterilization processing without requiring any special environment. The biotic indication device is constituted by a translucent outer container having an opening at one end and a closing sheet with which the opening of the outer container is closed, and which is permeable to steam but impermeable to germs. An inner container containing a compound for neutralizing hydrogen peroxide, an inner container containing a culture solution and a filter paper impregnated with a growable microorganism are housed in the outer container. One of the inner containers includes detection means which changes visually in response to the growth of the microorganism.

The external wall of the outer container is formed so as to be deformable but not easily breakable. The inner container containing a compound and the inner container containing a culture solution are formed of breakable members such as glass members.

When the biotic indication device is exposed to sterilizing steam, steam permeates through the closing seat and the microorganism in the filter paper housed in the outer container is exposed to the steam. After the completion of sterilization processing, the external wall of the outer container is deformed to break the inner container containing the compound and the inner container containing the culture solution. The filter paper is thereby immersed in the culture solution to grow the remaining microorganism by the culture solution. The sterilization effect can be checked according to whether or not the detection means changes visually in response to the growth of the microorganism.

Use of the biotic indication device enclosed in a test pack as well as use in a single state has been proposed. The test pack is for creating a situation where the biotic indication device is not easily exposed to sterilizing gas, and it is hard to perform sterilization thereon. The test pack simulates a situation where it is hardest to sterilize an object to be sterilized, i.e., a medical instrument. For example, the test pack has an absorption tool for inhibiting sterilizing gas from reaching on a passage from the opening through which sterilizing gas enters to the storage chamber in which the biotic indication device is placed. This means that the biotic indication device is disposed in a place where the biotic indication device is not easily exposed to sterilizing gas, and where it is hard to perform sterilization thereon.

Japanese Patent Laid-Open No. 7-148232, however, does not concretely disclose which portion of what medical instrument is hard to sterilize in a simulated situation of the test pack according to the invention.

There is an endoscope which has flexibility so as to be inserted in a body cavity and has an elongated insertion portion as one of medical instruments. The endoscope has a treatment instrument insertion tubular passage for inserting in the insertion portion a treatment instrument for treating an affected part in a body cavity, a forward water feed tubular passage for feeding water for cleaning an affected portion treated with the treatment instrument, a water/air feed tubular passage for feeding water and air to an observation window at the distal end of the insertion portion, and other portions. The tubular passages existing in the endoscope are small in inner diameter. It is, therefore, difficult to insert in the tubular passage the biotic indication device according to the Japanese Patent Laid-Open No. 7-148232.

If a biological indicator is formed into such a shape as to be insertable in the tubular passage provided in the endoscope, an operation to insert the biological indicator in the tubular passage before sterilization processing on the endoscope and an operation to draw the biological indicator out of the tubular passage after sterilization processing is performed. Also, there is a need to operate in a germ-free environment in order to prevent attachment of germs other than index germs when the biological indicator is inserted into or drawn out of the tubular passage and, hence, a need for a special environment in which a germ-free operation can be performed and operations by a skilled person.

The present invention has been achieved in consideration of the above-described circumstances and an object of the present invention is to provide an endoscope sterilization evaluation device which does not require a special environment and operations by a skilled person, and which is capable of reliably and easily checking the sterilization effect in a tubular passage provided in an insertion portion of an endoscope.

### Disclosure of the Invention

### Means for Solving the Problem

An endoscope sterilization evaluation device according to the present invention includes an elongated hollow body having a hollow portion opened at opposite ends, the elongated hollow body simulating an endoscope having a tubular passage, an exterior member having a first housing portion in which a biological indicator for performing sterilization evaluation on the hollow body is housed, and a second housing portion in which a culture solution for cultivating germs attached to the biological indicator is housed, the exterior member also having at a predetermined position a communication passage for communication between the first housing portion and the outside, and mount means for holding the communication passage in a state of communicating with the hollow portion, and mounting the exterior member to the hollow body.

### Brief Description of the Drawings

Fig. 1 is a diagram for illustrating a medium-integral-type biological indicator which is a sterilization evaluation container constituting an endoscope sterilization evaluation device according to an embodiment of the present invention;
Fig. 2 is a diagram for illustrating the relationship between an end constituent members constituting a hollow body, flexible tube members, a casing body and a housing case in an endoscope sterilization evaluation device according to a first embodiment of the present invention;
Fig. 3 is a sectional view for illustrating a hollow portion in the hollow body;
Fig. 4 is a sectional view for illustrating the configuration of a mount portion for mounting the sterilization evaluation container of the endoscope sterilization evaluation device;
Fig. 5 is a diagram for illustrating another configuration of the medium-integral-type biological indicator;
Fig. 6 is a sectional view for illustrating another configuration of the container connection portion constituting the endoscope sterilization evaluation device;
Fig. 7 is a sectional view for illustrating another configuration of the cover member of the endoscope sterilization evaluation device;
Fig. 8A is a diagram for illustrating another configuration of the medium-integral-type biological indicator;
Fig. 8B is a plan view seen from the cap side of the medium-integral-type biological indicator shown in Fig. 8A:
Fig. 9 is a sectional view showing another configuration of the mount portion to which the sterilization evaluation container shown in Fig. 8A constituting the endoscope sterilization evaluation device is mounted;
Fig. 10 is a sectional view showing still another configuration of the mount portion to which the sterilization evaluation container constituting the endoscope sterilization evaluation device is mounted;
Fig. 11 is a plan view seen from the holding rib side of the mount portion shown in Fig. 10;
Fig. 12 is a diagram illustrating still another configuration of the medium-integral-type biological indicator;
Fig. 13 is a sectional view showing the configuration of a mount portion to which the sterilization evaluation container shown in Fig. 12 constituting the endoscope sterilization evaluation device is mounted;
Fig. 14 is a sectional view for illustrating the configuration of a T-branch connection tube and a BI mount portion of an endoscope sterilization evaluation device according to a second embodiment of the present invention;
Fig. 15A is a sectional view for illustrating tubular passage holding ribs serving as tubular passage holding portion for holding the tubular passage body at predetermined intervals on the inner periphery of the flexible tube member constituting the endoscope sterilization evaluation device;
Fig. 15B is a sectional view for illustrating tubular passage holding portions provided on the flexible tube member as portions for holding the tubular passage body at predetermined intervals on the inner periphery of the flexible tube member constituting the endoscope sterilization evaluation device;
Fig. 16 is a sectional view for illustrating another configuration of the flexible tuber member constituting the endoscope sterilization evaluation device;
Fig. 17 is a sectional view for illustrating the entire configuration of an endoscope sterilization evaluation device according to a third embodiment of the present invention;
Fig. 18 is a sectional view for illustrating the relationship between the insertion portion member and the sterilization evaluation container constituting the endoscope sterilization evaluation device shown in Fig. 17;
Fig. 19 is an enlarged sectional view of a portion XIX in Fig. 18;
Fig. 20 is a perspective view for illustrating the configuration of a biological indicator incorporating index germ paper used in an endoscope sterilization evaluation device according to a fourth embodiment of the present invention;
Fig. 21 is a sectional view showing the configuration of the mount portion provided on the tubular passage member constituting the endoscope sterilization evaluation device according to the fourth embodiment of the present invention and the biological indicator mounted to the mount portion; and
Fig. 22 is a diagram, including a partial sectional view, for illustrating the configuration of an endoscope sterilization evaluation device according to a fifth embodiment of the present invention.

### Best Mode for Carrying Out the Invention

Embodiments of the present invention will be described in detail with reference to the drawings.

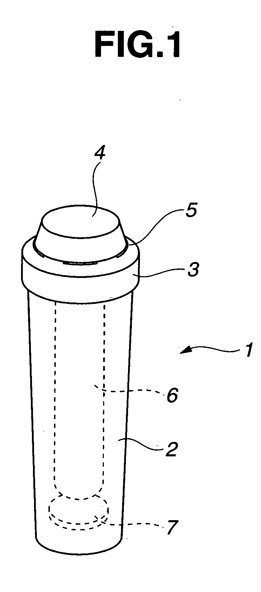

An endoscope sterilization evaluation device according to the first embodiment of the present invention will be described with reference to Figs. 1 through 13.

Description will first be made of a medium-integral-type biological indicator which is an exterior member constituting the endoscope sterilization evaluation device according to the embodiment of the present invention (hereinafter referred to as a sterilization evaluation container).

The sterilization evaluation container typically houses, in one container, paper dried after applying index germs thereto and a capsule in which a culture solution used to cultivate the index germs is encapsulated. Fig. 1 shows a concrete configuration thereof.

As shown in Fig. 1, the integral-type BI 1 is constituted by a container 2, a head portion 3, a cap 4, index germ paper 7, and a culture solution capsule 6.

The container 2 is a transparent member formed into a generally cylindrical shape. The head portion 3 is provided so as to cover an opening of the container 2. The head portion 3 has a central through hole and is formed so as to have an outside diametric size larger than the outside diameter of the container 2. The cap 4 is attached on the central through hole. The head portion 3 is provided with a plurality of sterilizing gas penetration holes 5 along the outer periphery of the cap 4 attached on the central through hole. The sterilizing gas penetration holes 5 are formed so as to be penetrable to sterilizing gas but impenetrable to index germs, a culture solution and the like. The index germ paper 7 is a biological indicator in which index germs such as spores or microorganisms are attached or impregnated to a medium such as paper or cloth and dried. The culture solution capsule 6 is a capsule in which a culture solution for cultivating the index germs on the index germ paper 7 is encapsulated.

The container 2 and the head portion 3 are formed separately from each other and are configured so that the head portion 3 is fitted to the opening end of the container 2 in an airtight manner. Alternatively, the container 2 and the head portion 3 are integrally formed of the same member. The head portion 3 also functions as a grasping portion to be held by an operator when the operator handles the integral-type BI 1.

The cap 4 is attached on the head portion 3 after the index germ paper 7 and the culture solution capsule 6 were housed in the container 2. Separation between the inside and the outside of the container 2 is thereby achieved. However, only sterilizing gas penetrates into the container 2 through the sterilizing gas penetration holes 5 provided in the head portion 3.

The container 2 is formed of a comparatively flexible member. Therefore, the container 2 is deformed without being broken when a side surface portion of the container is inwardly pressed. In contrast, the culture solution capsule 6 is formed of a glass member or the like comparatively easily breakable by external force. The culture solution encapsulated in the culture solution capsule 6 contains a substance which changes the color of the solution according to the cultivated condition of the index germs. Further, the container 2 and the cap 4 as a lid member form a housing portion in which the index germ paper 7 as a biological indicator is housed, and which is provided with the culture solution capsule 6 as a culture solution housing member having a culture solution encapsulated therein. That is, the container 2 and the cap 4 form a housing portion in which the culture solution capsule 6 and the index germ paper are placed.

When sterilization processing is performed on the integral-type BI 1, i.e., the sterilization evaluation container, by setting the integral-type BI 1 in a chamber of a sterilization unit, sterilizing gas penetrates into the container 2 through the sterilizing gas penetration holes 5 provided in the head portion 3. The index germs attached to the index germ paper 7 is thereby exposed to the sterilizing gas. After sterilization processing, the user breaks the culture solution capsule 6 in the container by pressing and deforming a side surface portion of the container 2, thereby causing the culture solution leaking to the interior of the container 2. The index germ paper 7 is thereby immersed in the culture solution. Thereafter, the user sets in a cultivation unit not shown in the figure the container 2 in which the index germ paper 7 immersed in the culture solution is housed, and performs cultivation at a predetermined temperature for a predetermined time period. After the lapse of the predetermined time period, the user checks the destroyed condition of the index germs of the index germ paper 7 from a change in color of the culture solution. The user checks and determines the operating condition of the sterilization unit based on the checked result. If this integral-type BI 1 is used, there is no need for any germ-free operation at the time of cultivation of the index germs. Thus, the needs to provide any special piece of equipment and employ a skilled person are eliminated.

In a case where determination of the sterilized state of a tubular passage formed in an endoscope is made by means of the above-described integral-type BI 1, it is difficult to insert the integral-type BI 1 in the tubular passage in the insertion portion of the endoscope unless the outside diameter of the integral-type BI 1 is set equal to or smaller than the inner diameter of the tubular passage. Moreover, in a case where the integral-type BI 1 is formed so that its size is equal to or smaller than the inner diameter of the tubular passage, an operation to insert the integral-type BI 1 in the tubular passage before sterilization processing and an operation to draw the integral-type BI 1 out of the tubular passage after sterilization processing are required and the operations become complicated.

Then, according to the present invention, a tubular passage in an endoscope is simulated and an endoscope sterilization evaluation device capable of enabling the above-described integral-type BI 1 to be easily attached in and removed from the tubular passage is configured. In the endoscope sterilization evaluation device, sterilization is performed by putting the endoscope sterilization evaluation device and the endoscope together in a chamber of a sterilization unit. Thereafter, the destroyed condition of the index germs in the integral-type BI 1 constituting the endoscope sterilization evaluation device is checked to determine the sterilized condition of the endoscope.

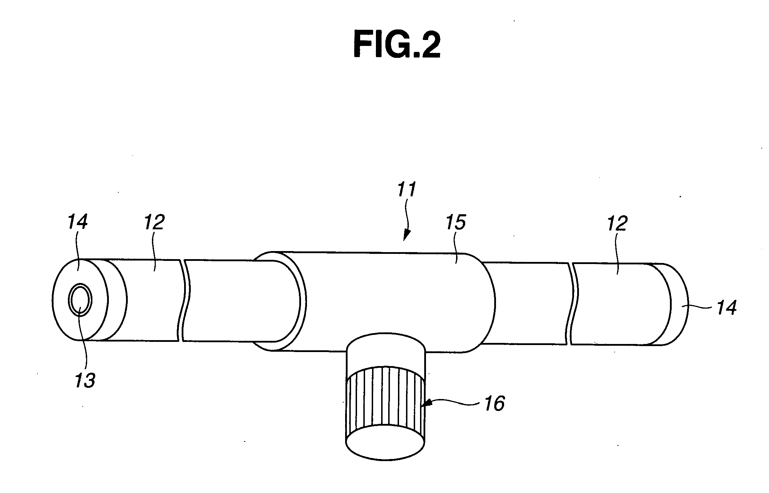

The exterior of an endoscope sterilization evaluation device 11 according to the first embodiment of the present invention is constituted by, as shown in Fig. 2, a pair of flexible tube members 12 each formed of a tubular flexible member, a pair of end constituent members 14 disposed at openings in the flexible tube members 12 at one ends of the same and having through holes generally at their centers, a case main body 15 having its ends placed at each of the other ends of the flexible tube members 12, and a housing case 16 in which the integral-type BI 1 is enclosed. The flexible tube members 12, the end constituent members 14, the case main body 15 and the housing case 16 form a hollow body having an internal space 11A.

The flexible tube members 12 and the case main body 15 simulate the exterior of a flexible portion of an endoscope. One end of a tubular passage body 13 having a hollow simulating a tubular passage in the endoscope is connected to each of the through holes of the end constituent members 14. A pair of the tubular passage bodies 13 are provided and are respectively inserted in the flexible tube members 12. Thus, hollow bodies having hollows therein are formed.

The housing case 16 is connected to a side surface of the case main body 15. In the present embodiment, the endoscope sterilization evaluation device 11 is formed into a shape bilaterally symmetrical about the case main body 15 by setting the lengths of the flexible tube members 12 equal to each other.

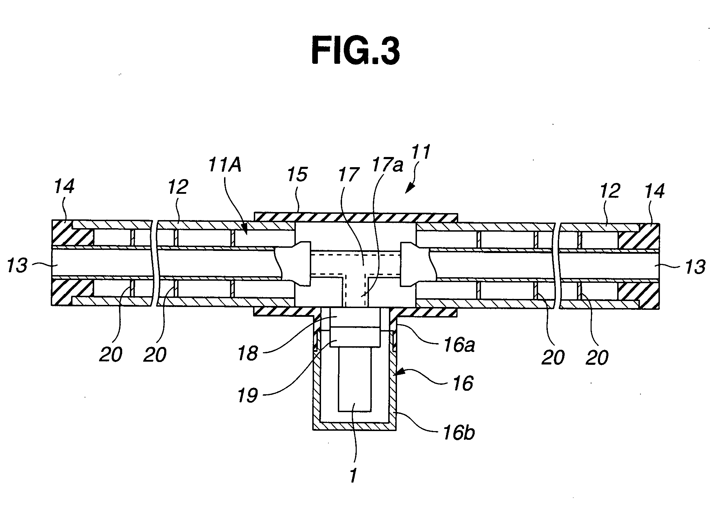

The internal configuration of the endoscope sterilization evaluation device 11 will be described with reference to Fig. 3.

The tubular passage bodies 13 are inserted in the flexible tube members 12 along the axis in the lengthwise direction. The tubular passage bodies 13 are held on the inner periphery of the flexible tube members 12 by a plurality of tubular passage holding ribs 20 disposed at predetermined intervals. The tubular passage holding ribs 20 hold the tubular passage bodies 13 in a spaced positional relationship with the inner walls of the flexible tube members 12, such that the tubular passage bodies 13 do not contact the inner walls. Heat conducted to the flexible tube members 12 during autoclave sterilization processing is thereby prevented from being directly conducted to the tubular passage bodies 13.

A T-branch connection tube 17 having a branch tube 17a and constituting a hollow is provided in the case main body 15. The other ends of the tubular passage bodies 13 are connected to the tube portions other than the branch tube 17a constituting the T-branch connection tube 17. The T-branch connection tube 17 is for branching off each tubular passage body 13 to the housing case 16 side of the case main body 15. The branch tube 17a of the T-branch connection tube 17 is placed in the housing case 16 direction from the case main body 15.

A supplementary description will be made of the tubular passage bodies 13 including the T-branch connection tube 17. The internal tubular passage formed as a hollow formed by the tubular passage bodies 13 and the T-branch connection tube 17 simulates the tubular passage in the endoscope. The inner diameter and the length of the internal tubular passage is set to a size obtained as a combination of an inner diameter and a length corresponding to the conditions of a place in the endoscope where it is hardest to sterilize the endoscope. More specifically, a setting is made according to the conditions of a central portion, which is a place where it is hardest to perform sterilization. Accordingly, the T-branch connection tube 17 is provided at a center of the tubular passage bodies 13 to enable the branch tube 17a to connect to the integral-type BI 1. That is, the entire length of the tubular passage bodies 13 with the T-branch connection tube 17 provided at the center is set equal to the length in the above-described endoscope tubular passage at which it is hardest to perform sterilization. The inner diameters of the T-branch connection tube 17 and the tune passage bodies 13 are equal to each other and set equal to the inner diameter in the endoscope tubular passage at which it is hardest to perform sterilization.

The housing case 16 is constructed by mounting and fixing a cover member 16b to an attachment portion 16a extending from the side surface of the case main body 15. A mount portion 18 which is mount means is provided on a distal end portion of the branch tube 17a of the T-branch connection tube 17. A container connection portion 19 constituting mount means housing the integral-type BI 1 is detachably mounted on the mount portion 18. That is, the integral-type BI 1 is detachably mounted to the branch tube 17a of the T-branch connection tube 17 by means of the mount portion 18 and the container connection portion 19. The cover member 16b is placed so as to cover the outer periphery of the integral-type BI 1 mounted by means of the mount portion 18 and the container connection portion 19. The cover member 16b is airtightly attached to the attachment portion 16a.

The connection portions of the flexible tube members 12 and the case main body 15 and the connection portions of the opposite ends of the flexible tube member 12 and the end constituent members 14 are airtightly bonded and fixed. Also, the connection portions of the tubular passage bodies 13 and the end constituent members 14 are airtightly bonded and fixed. The interior of each flexible tube member 12 is thereby maintained in an airtight state.

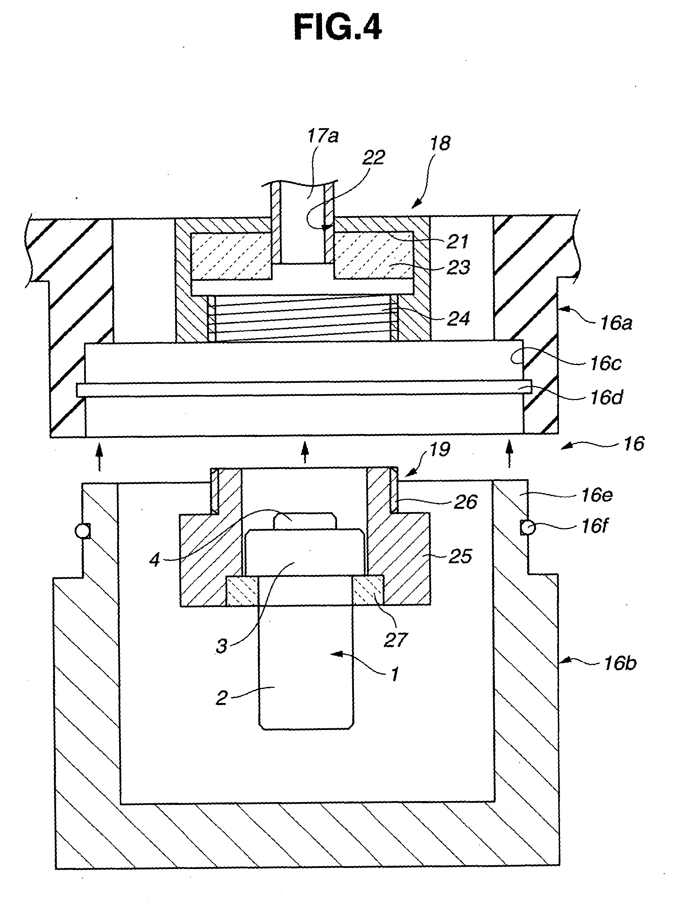

The configuration of the housing case 16, the mount portion 18 and the container connection portion 19 will be described with reference to Fig. 4.

The attachment portion 16a constituting the housing case 16 projects in tubular form from the side surface of the case main body 15. The cover member 16b is tubular and its opening side is attached to the attachment portion 16a. The branch tube 17a as a branch passage of the T-branch connection tube 17 extends in the attachment portion 16a. A female threaded portion 16c with which a male threaded portion 16e provided on the cover member 16b is screw-engaged and an O-ring groove 16d to which an O-ring 16f is airtightly fitted are provided in the inner peripheral surface of the attachment portion 16a. The male threaded portion 16e is formed on the outer periphery of an opening-side end portion of the cover member 16b. The O-ring 16f is provided so as to position on the base end side relative to the male threaded portion 16e. Therefore, as the male threaded portion 16e of the cover member 16b is screw-engaged with the female threaded portion 16c of the attachment portion 16a, the O-ring 16f of the cover member 16b is tightly fitted in the O-ring groove 16d of the attachment portion 16a. In this way, the interior of the case main body 15 can be made airtight. The O-ring 16f is an elastic member made of silicon or the like.

The mount portion 18 is a cylindrical member having a recess. A through hole 22 is provided in a bottom surface 21 at a center of the same. A female threaded portion 24 is provided in an opening inner peripheral surface on the container mount side. The branch tube 17a constituting the T-junction 17 is inserted and mounted in the through hole 22. A backing member 23 formed of an elastic member in annular form made of silicon or the like is placed around the through hole 22 in the mount portion 18. The branch tube 17a is airtightly bonded to the through hole 22 of the mount portion 18. The backing member 23 is formed and placed so as not to close the opening of the branch tube 17a.

The container connection portion 19 is constituted mainly by a connection portion main body 25 and a fastening ring 27. The connection portion main body 25 has a stepped tubular shape and a through hole formed at a center. The head portion 3 of the integral-type BI 1 is placed in the through hole. A male threaded portion 26 to be screw-engaged with the female threaded portion 24 of the mount portion 18 is provided on the outer periphery of the connection portion main body 25. A large-diameter recessed portion is provided on the base end side of the through hole. The fastening ring 27 in annular form is disposed in the recessed portion. The inner diameter of the fastening ring 27 is smaller than the outside diameter of the container 2. The material forming the fastening ring 27 has elasticity. When the container 2 is placed in the through hole of the fastening ring 27, the inner peripheral surface of the through hole is brought into close contract with the outer peripheral surface of the container 2 to be airtightly fastened and held. The fastening ring 27 is airtightly bonded and fixed in the recessed portion of the connection portion main body 25. Accordingly, the container 2 of the integral-type BI 1 is mounted in the container connection portion 19 from its bottom side in the top-to-bottom direction as viewed in the figure. Since the outside diameter of the container 2 portion is larger than the inner diameter of the fastening ring 27, the container 2 is set in a state of being press-fitted in the fastening ring 27. The container 2 is fastened and held with the fastening ring 27 to maintain airtightness between the inner periphery of the connection portion main body 25 and the container 2 of the integral-type BI 1.

The container connection portion 19 in which the integral-type BI 1 is mounted is mounted to the mount portion 18, as indicated by the arrow in the figure. The male threaded portion 26 of the container connection portion 19 and the female threaded portion 24 of the mount portion 18 are then screwed-engaged with each other. As the container connection portion 19 is screw-engaged with the mount portion 18, the top end surface of the connection portion main body 25 constituting the container connection portion 19 is brought into close contact with the backing member 23 provided on the mount portion 18. The interior of the mount portion 18 and the interior of the container connection portion 19 are thereby made airtight while providing communication between the tubular passage bodies 13 and the sterilizing gas penetration holes provided in the head portion 3 of the integral-type BI 1 through the T-branch connection tube 17.

After the integral-type BI 1 was mounted by means of the mount portion 18 and the container connection portion 19, the cover member 16b is screw-engaged with the attachment portion 16a to cover the outer periphery of the integral-type BI 1, as indicated by the arrow in the figure. The outer periphery of the integral-type BI 1 is thereby housed in state where airtight communication from the inside of the housing case 16 to the inside of each flexible tube member 12 is provided.

Thus, the tubular passage bodies 13, the T-branch connection tube 17, the mount portion 18, the container connection portion 19 and the integral-type BI 1 are airtightly covered with the flexible tube members 12, the case main body 15, the end constituent members 14 and the housing case 16. In the resulting structure, the heat cannot be easily conducted from the outside directly to the tubular passage bodies 13, the T-branch connection tube 17, the mount portion 18, the container connection portion 19 and the external surface of the integral-type BI 1.

The endoscope sterilization evaluation device 11 thus configured is placed in a chamber of a sterilization unit together with an endoscope device to be sterilized, and sterilization processing is performed under predetermined sterilization processing conditions. When the chamber of the sterilization unit is filled with sterilizing gas for example, the sterilizing gas also penetrates into the internal tubular passage of the endoscope to sterilize the interior of the tubular passage. Also, the sterilizing gas penetrates into the tubular passage bodies 13 via the openings at the opposite ends of the flexible tube members 12 of the endoscope sterilization evaluation device 11. The sterilizing gas penetrates into the container 2 from the branch tube 17a of the T-branch connection tube 17 by passing through the sterilizing gas penetration holes 5 provided in the upper surface of the head portion 3 of the integral-type BI 1.

In achieving sterilization, the temperature is also an important factor. The tubular passage inner periphery of the endoscope is increased in temperature according to the temperature of the penetrating sterilizing gas. On the other hand, constituents of the endoscope (e.g., the flexible portion of the endoscope) exist around the tubular passage in the endoscope. Therefore, conduction of heat from the external surface of the endoscope to the tubular passage inner periphery is not easily effected in comparison with the inner surface side directly heated by the sterilizing gas. That is, heating through the direct contact of the sterilizing gas is dominant in conduction of heat to the tubular passage inner peripheral surface (but the amount of heat conducted from the external surface of the endoscope to the tubular passage inner peripheral surface is not zero).

As described above, in the endoscope sterilization evaluation device 11, the tubular passage bodies 13, which are internal tubular passages, the T-branch connection tube 17, the mount portion 18, the container connection portion 19 and the integral-type BI 1 are airtightly covered with the flexible tube members 12, the case main body 15, the end constituent members 14 and the housing case 16. Thus, the structure is such that direct conduction of heat from the external surface of the endoscope sterilization evaluation device 11 to the inner periphery of the internal tubular passage is not easily effected (external surface → air → external surface of internal tubular passage → inner periphery of internal tubular passage), as in the actual endoscope. That is, the endoscope sterilization evaluation device 11 also simulates the way in which heat is conducted from the external surface to the tube inner periphery in the actual endoscope device.

The index germ paper 7 placed in the container 2 is exposed to the sterilizing gas that has penetrated into the container 2 of the integral-type BI 1.

After the completion of sterilization by the sterilization unit on the endoscope and the endoscope sterilization evaluation device 11, the user takes the endoscope sterilization evaluation device 11 out of the sterilization unit. The user also detaches the cover member 16b of the housing case 16 from the attachment portion 16a. The user then removes the container connection portion 19 from the mount portion 18. Thereafter, the user draws the integral-type BI 1 out of the through hole of the connection portion main body 25 of the container connection portion 19 by the procedure reverse to that at the time of mounting. The user then presses and deforms a side surface of the container 2 constituting the integral-type BI 1 to break the culture solution capsule 6 therein. The culture solution then leaks out of the culture solution capsule 6 and the index germ paper 7 is immersed in the culture solution. Subsequently, the integral-type BI 1 in the state where the culture solution capsule 6 is broken and the index germ paper 7 is immersed in the culture solution is kept at a predetermined temperature for a predetermined time period in a heating unit not shown in the figure. During this time period, if index germs remain on the index germ paper 7, they are cultivated to change the color of the culture solution. If the index germs on the index germ paper 7 have died, the culture solution does not change in color. That is, in a case where all the index germs on the index germ paper 7 have died, the user determines that sterilization processing has been performed sufficiently effectively. In a case where the culture solution has changed in color, the user determines that the index germs on the index germ paper 7 remain and, therefore, that sterilization processing has not been performed sufficiently effectively.

That is, the effect of sterilization of the tubular passage in the endoscope can be determined from the result of cultivation of index germs after sterilization processing on the integral-type BI 1 provided in the endoscope sterilization evaluation device 11 set under generally the same conditions as the tubular passage conditions under which it is hardest to sterilize the endoscope tubular passage. Moreover, since the integral-type BI 1 can be used, the need for the operations to take out the index germ paper 7 in a germ-free manner after sterilization processing and cultivate the germs is eliminated. More specifically, only the operations to break the culture solution capsule 6 incorporated in the container 2 of the integral-type BI 1 from the outside of the container 2 and perform warming thereon by a heating unit are performed. It is, therefore, possible to easily and reliably determine the result of sterilization processing without requiring a special environment and a skilled person for handling index germs in a germ-free manner.

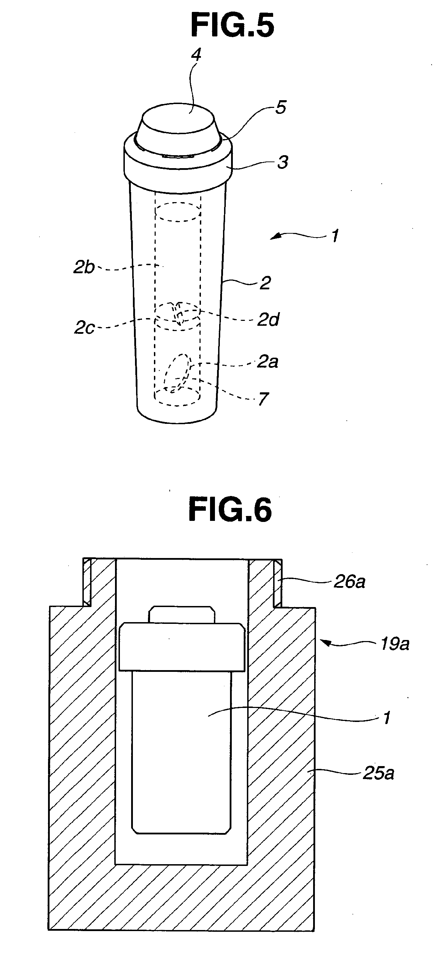

The configuration of the integral-type BI 1 is not limited to that in which the culture solution is encapsulated in the culture capsule 6 as shown in Fig. 1. For example, a configuration is conceivable in which, as shown in Fig. 5, an index germ paper housing portion 2a as a first housing portion in which index germ paper 7 is housed and a culture solution housing portion 2b as a second housing portion containing a culture solution in a sealing manner are provided in the container 2. In such a case, a cut 2d for forming a smaller-thickness portion is provided in a partition wall portion 2c for portioning between the index germ paper housing portion 2a and the culture solution housing portion 2b. The container 2 is formed of a resin, and a force is externally applied to the container 2 to break the partition wall portion 2c from the cut 2d, thereby immersing the index paper in the culture solution.

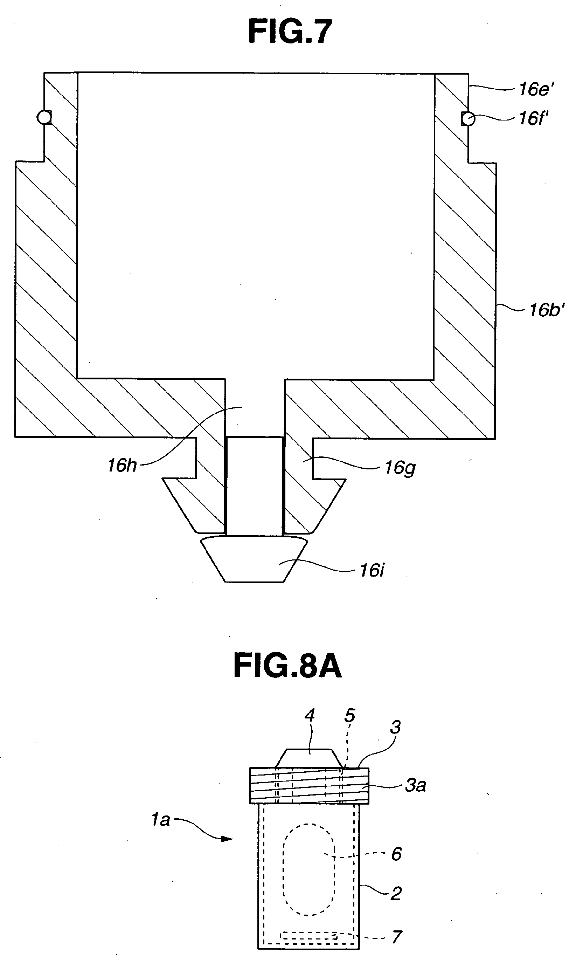

Modified examples of the housing case of the endoscope sterilization evaluation device according to the present invention will be described with reference to Figs. 6 and 7.

As shown in Fig. 6, a container connection portion 19a configured as mount means disposed in the housing case 16 is a connection portion main body 25a in tubular form having a recess. The whole of the integral-type BI 1 is housed in the recess of the connection portion main body 25a. A male threaded portion 26a to be screw-engaged with the female threaded portion 24 of the mount portion 18 is formed on the outer periphery of the connection portion main body 25a on the recess opening side.

That is, the container connection portion 19a having the recess in which the whole of the integral-type BI 1 is housed is formed. The integral-type BI 1 is housed in the recess of the container connection portion 19a and the male threaded portion 26a of the container connection portion 19a and the female threaded portion 24 of the connection member 18 are screw-engaged with each other. Then the integral-type BI 1 housed in the container connection portion 19a communicates with the tubular passage bodies 13 via the T-branch connection tube 17. If this container connection portion 19a is used, the number of component parts can be reduced in comparison with the above-described container connection portion 19 to achieve a low-priced configuration.

As shown in Fig. 7, an airtightness detection ferrule 16g is provided on a cover member 16g' constituting the housing case 16. A through hole 16h which provides communication between the cover member internal space and the outside is formed in the airtightness detection ferrule 16g. Other configuration is the same as that of the cover member 16b described with reference to Fig. 4. The same members are indicated by the same reference characters and the description for them will not be repeated.

An airtightness detection device not shown in the figure can be connected to the airtightness detection ferrule 16g. Also, the airtightness detection ferrule 16g is closed by an airtight cap 16i when the airtightness detection device is not connected. The airtight cap 16i formed of an elastic member. The outside diameter of the portion inserted in the through hole 16h is larger than the inner diameter of the through hole 16h. Accordingly, the airtight cap 16i is press-fitted and fixed in the through hole 16h to maintain airtightness.

A use method in a case where the cover member 16b' described with reference to Fig. 7 is used in place of the cover member 16b shown in Fig. 3 will be described.

Before the endoscope sterilization evaluation device 11 is put in the sterilization unit, the airtight cap 16i is removed and the airtightness detection device is connected to the airtightness detection ferrule 16g. Thereafter, compressed air for example is supplied from the airtightness detection device to the internal space in the cover member 16' via the through hole 16h. The compressed air supplied to the internal space in the cover member 16' is supplied from the inner hole of the attachment portion 16a to the internal space 11 A formed by portions including the inner peripheries of the flexible tube members 12 and the outer peripheries of the tubular passage bodies 13. In case of leakage of air to the outside when compressed air is being supplied, it can be understood that a crack exists in any of the flexible tube members 12, the tubular passage bodies 13, the case main body 15 and the T-branch connection tube 17 or any of the airtight connections therebetween is broken. If such a fault occurs, sterilizing gas also intrudes from the airtight connection broken portion other than the openings of the tubular passage bodies 13 provided at the opposite ends of the flexible tube members 12. Then the sterilizing gas can penetrate into the integral-type BI 1 more easily in comparison with the case of penetration via the proper tubular passage. Direct contact of sterilizing gas with the external surfaces of the tubular passage bodies 13 relaxes the heat conduction conditions. Thus, the provision of the cover member 16b' makes it possible to detect, before sterilization processing, whether or not the endoscope sterilization evaluation device 11 is in the normal state. Also, if airtightness detection is executed after sterilization processing, it is possible to detect whether or not a fault has occurred, for example, in the airtight connections during sterilization processing.

Thus, the reliability of the result obtained from the integral-type BI 1 can be improved by providing the airtightness detection ferrule.

Another modified example of the housing case of the endoscope sterilization evaluation device according to the present invention will be described with reference to Figs. 8A to 9.

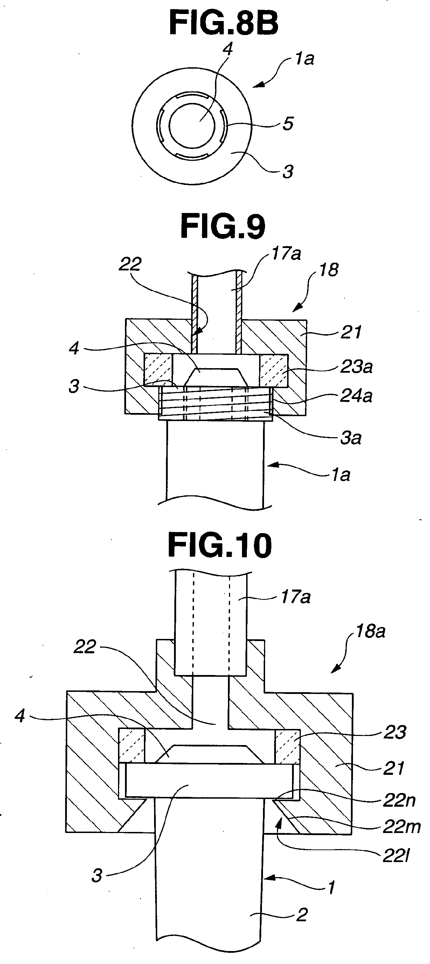

An integral-type BI 1a shown in Figs. 8A and 8B are configured so as to be capable of being directly mounted to the mount portion 18. As shown in Fig. 8B, the integral-type BI 1a is provided with a male threaded portion 3 a constituting mount means on the outer periphery of the head portion 3. The configuration other than the head portion 3, i.e., the configuration of the container 2, the cap 4, the sterilizing gas penetration holes 5 provided in the upper surface of the head portion 3, the culture solution capsule 6 and the index germ paper 7 is the same as that shown in Fig. 1.

On the other hand, in the mount portion 18, as shown in Fig. 9, a female threaded portion 24a constituting mount means screw-engaged with the male threaded portion 3a on the head portion 3 of the integral-type BI 1a is provided on a mount main body 21. The branch tube 17a of the T-branch connection tube 17 is mounted in the through hole 22. The annular backing member 23a provided on the mount portion 18 is formed into such a shape as to avoid closing the sterilizing gas penetration holes 5. Therefore, when the male threaded portion 3a on the head portion 3 constituting the integral-type BI 1a is screw-engaged with the mount main body 21 to place the upper surface of the head portion 3 constituting the integral-type BI 1a in close contact with the backing member 23a, sterilizing gas penetrates into the container through the sterilizing gas penetration holes 5.

In this way, the integral-type BI 1a can be mounted to the mount portion 18 without using the above-described container connection portion 19 or container connection portion 19a. Therefore an endoscope sterilization evaluation device of a lower price can be configured in comparison with the case of using the container connection portion 19a.

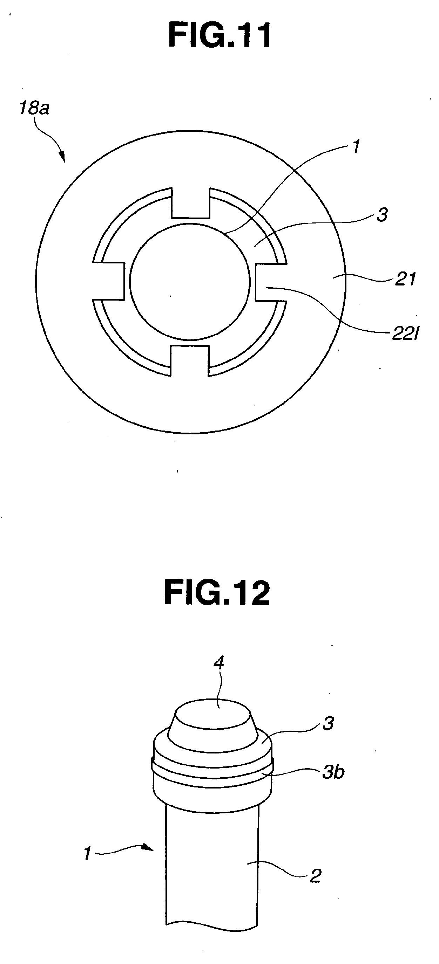

Still another modified example of the housing case of the endoscope sterilization evaluation device will be described with reference to Figs. 10 and 11.

In the present modified example, the integral-type BI 1 shown in Fig. 1 or 5 is used and a mount portion 18a shown in Fig. 10 is used in place of the mount portion 18 described above with reference to Figs. 3 and 4. The mount portion 18a is constituted by a mount main body 21 having a through hole 22 and holding ribs 221 and an annular backing member 23.

The branch tube 17a of the T-branch connection tube 17 is inserted and fixed in the through hole 22. The holding ribs 221 are formed on the inner periphery of the mount portion main body 21.

As shown in Fig. 11, the holding ribs 221 are formed so as to project from the inner peripheral surface of the mount opening to a center. The holding ribs 221 are provided at intervals of about 90 degrees on the inner periphery of the mount opening. The head portion 3 of the integral-type BI 1 is inserted in the mount portion main body 21 from below, as shown in Fig. 10. Therefore the holding ribs 221 have slanting portions 22m and engaging portions 22n. The slanting portions 22m are brought into contact with a side surface of the head portion 3 to deform the head portion 3. The engaging portions 22n are placed on the lower side of the head portion 3 to place the head portion 3 on the backing member 23 in a pinching manner. That is, the integral-type BI 1 is inserted along the slanting portions 22m of the holding ribs 221 from the opening side of the mount portion main body 21. Then the holding ribs 221 are deformed and placed below the head portion 3 of the integral-type BI 1. Airtightness is thereby maintained between the backing member 23 and the engaging portions 22n of the holding ribs 221. The through hole 22 of the mount portion main body 21 and the branch tube 17a are airtightly connected to each other. The backing member 23 is formed and placed so as not to close the sterilizing gas penetration holes 5 of the integral-type BI 1.

Thus, the integral-type BI 1 can be mounted to the mount portion 18a by a single operation and the integral-type BI 1 can be airtightly connected to the T-branch connection tube 17 from which the tubular passage bodies 13 diverge. In this way, the mounting handling of the integral-type BI 1 is improved. That is, the integral-type BI 1 is mounted by the mount portion main body 21 having the holding ribs 221, as mount means, to be engaged with the engaging portions 22n of the head portion 3.

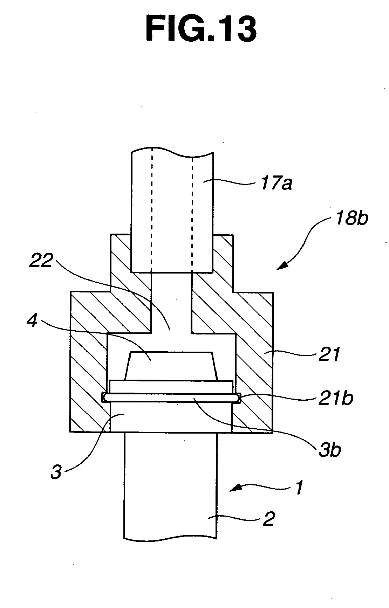

The yet another fourth modified example of the housing case of the endoscope sterilization evaluation device according to the present invention will be described with reference to Figs. 12 and 13.

In the present modified example, as shown in Fig. 12, an O-ring 3b is provided as mount means on the outer periphery of the head portion 3 of the integral-type BI 1 described with reference to Fig. 1 or 5. On the other hand, as shown in Fig. 13, an O-ring groove 21 b constituting mount means is provided in the mount portion main body 21 constituting the mount portion 18b. The O-ring 3b formed in the inner peripheral surface of the hole portion in which the outside diameter of the head portion 3 of the integral-type BI 1 is inserted and provided in the head portion 3 of the integral-type BI 1 is airtightly fitted in the O-ring groove 21b.

That is, the head portion 3 of the integral-type BI 1 is mounted in the opening in the mount portion main body 21 of the mount portion 18b, with the O-ring 3b fitted in the O-ring groove 21b in the mount portion main body 21. The integral-type BI1 is thereby held airtightly in the opening of the mount portion main body 21. That is, the integral-type BI 1 is mounted by the mount portion main body 21 having the O-ring groove 21b as mount means.

In this way, the integral-type BI 1 can be mounted in the mount portion 18b by a single operation. Also, the integral-type BI 1 can be airtightly connected to the T-branch connection tube 17 from which the tubular passage bodies 13 diverge, thus improving handling.

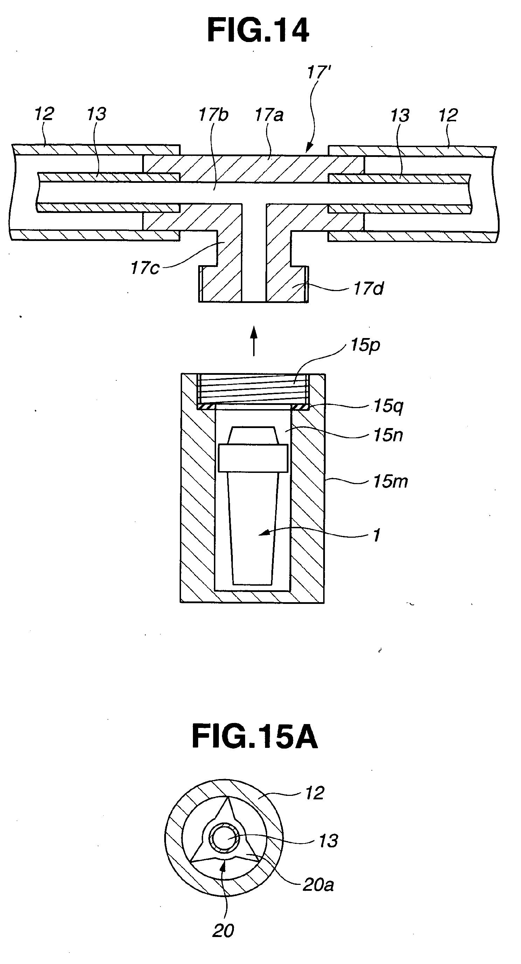

An endoscope sterilization evaluation device according to a second embodiment of the present invention will be described with reference to Fig. 14.

Points of difference between the endoscope sterilization evaluation device according to the present embodiment and the endoscope sterilization evaluation device according to the above-described first embodiment will be described. The endoscope sterilization evaluation device according to the second embodiment differs in the configuration of the T-branch connection tube 17 and the case main body 15 constituting the endoscope sterilization evaluation device according to the first embodiment described above with reference to Fig. 2.

As shown in the figure, a branch connection tube 17' is a thick tube formed into the shape of T by a cylindrical body 17a and a branch portion 17c. Flexible tube members 12 are respectively connected airtightly to the outer peripheries of opposite end portions of the cylindrical body 17a. Tubular passage bodies 13 communicate with the opposite end portions of the cylindrical body 17a at centers thereof. The cylindrical body 17a has a tubular passage portion 17b about its center axis. The inner diameter of the tubular passage portion 17b and the inner diameter of the tubular passage bodies 13 are equal to each other. The branch portion 17c extends from a central side surface of the cylindrical body 17a in a direction perpendicular to the axis. The branch portion 17c has a tubular passage communicating with the tubular passage portion 17b. The branch portion 17c has a male threaded portion 17d as mount means on the outer peripheral surface of its end portion. That is, the branch connection tube 17' is T-shaped and has tubular passages extending in three directions.

The branch connection tube 17' having the cylindrical body 17a and the branch portion 17c is formed of a member exhibiting the same heat conduction as the heat conduction between the flexible tube members 12 and the tubular passage bodies 13. That is, the second embodiment is constituted by the flexible tube members 12 which are a substitute for a flexible portion of an endoscope, the tubular passage bodies 13 which are members simulating the tubular passage in the endoscope, and the thick T-shaped branch connection tube 17' provided at a center of the tubular passage bodies 13.

On the other hand, the integral-type BI 1 is housed in a cylindrical housing container 15m having a housing chamber 15n in which the integral-type BI 1 is housed. A recessed portion is provided on the opening side of the housing container 15m. A female threaded portion 15p is formed as mount means in the inner peripheral surface of the recessed portion, and an O-ring 15q is interposed on a bottom portion. The female threaded portion 15p is screw-engaged with the male threaded portion 17d of the branch connection tube 17'. The wall thickness of the housing container 15m is set so that the heat conduction from the outside to the interior of the housing chamber 15n is same, as in the case of the branch connection tube 17'. In other respects, the configuration and advantages are the same as those of the above-described first embodiment. That is, the integral-type BI 1 is mounted with the male threaded portion 17d constituting mount means and the housing container 15m having the female threaded portion 15p.

This configuration makes it possible to eliminate the case body 15 and the housing case 16. Also, the housing container 15m in which the integral-type BI 1 is housed can be directly mounted to the branch connection tube 17' with a simple configuration. A low-priced endoscope sterilization evaluation device can therefore be provided.

Tubular passage holding portions for holding the tubular passage bodies placed on the inner periphery of the flexible tube members of the endoscope sterilization evaluation device at predetermined intervals will be described with reference to Figs. 15A to 16.

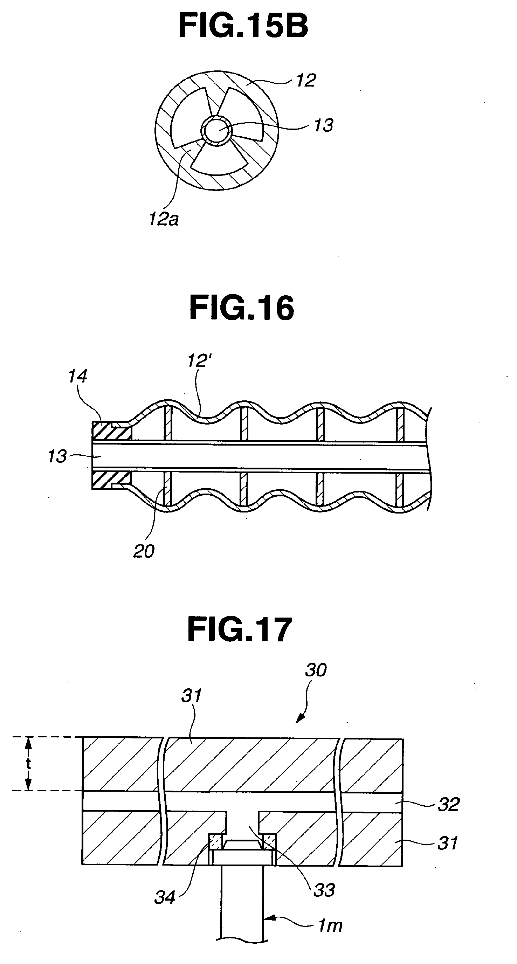

A plurality of tubular passage holding portions for holding the tubular passage bodies 13 disposed on the inner periphery of the flexible tube members 12 of the endoscope sterilization evaluation device will be described with reference to Figs. 15A and 15B.

The tubular passage holding portions maintains the positional relationship between the flexible tube members 12 simulating the endoscope flexible portion and the tubular passage bodies 13 simulating the tubular passage in the endoscope.

As shown in Fig. 15A, the tubular passage holding portions are supporting ribs 20a provided on each tubular passage holding rib 20. The tubular passage holding rib 20 has a fixed portion 20b mounted and fixed on the outer periphery of the tubular passage body 13. The supporting ribs 20a of the tubular passage holding rib 20 are placed in contact with the inner peripheral surface of the flexible tube member 12. The supporting ribs 20a project from the outer periphery of the fixed portion 20b, for example, in three directions at intervals of about 120 degrees or in four directions, not shown in the figure, at intervals of about 90 degrees.

As shown in Fig. 15B, supporting ribs 12a may alternatively be provided as tubular passage holding portions on the flexible tube member 12. The supporting ribs 12a are placed in contact with the outer peripheral surface of the tubular passage body 13. The supporting ribs 12a project from the inner periphery of the flexible tube member 12, for example, in three directions at intervals of about 120 degrees or in four directions, not shown, at intervals of about 90 degrees.

Thus, the tubular passage body 13 is placed with a predetermined spacing from the inner periphery of the flexible tube member 12. In this way, the conditions for heat conduction from the flexible tube member 12 to the tubular passage body 13 can be made uniform.

A bellows-like flexible tube member 12' obtained by changing the external shape of the flexible tube member 12 into a bellows-like shape as shown in Fig. 12 may alternatively be used. The tubular passage body 13 inserted in the bellows-like flexible tube member 12' is held with a predetermined spacing by tubular passage holding ribs 20. The bellows-like flexible tube member 12' is sturdy against a bending operation. Therefore, when the bellows-like flexible tube member 12' is placed in a curved state in a chamber of a sterilization unit, the positional relationship between the bellows-like flexible tube member 12' and the tubular passage body 13 can be maintained more reliably.

In this way, the tubular passage bodies 13 of the endoscope sterilization evaluation device 11 are held with a predetermined spacing without contacting the inner peripheral surfaces of the flexible tube members 12 or 12" when placed in the sterilization unit. It is, therefore, possible to prevent heat in the flexible tube members 12 from being conducted to the tubular passage bodies 13.

An endoscope sterilization evaluation device according to a third embodiment of the present invention will be described with reference to Figs. 17 to 19.

As shown in Fig. 17, an endoscope sterilization evaluation device 30 according to the present embodiment is an insertion portion main body 31 formed of a flexible cylindrical body simulating the entire shape of a flexible portion of an endoscope. The insertion portion main body 31 has a tuber path hole 32 formed at a center along the axial direction so as to simulate a tubular passage in an endoscope.

The insertion portion main body 31 has in a side surface at a center in its lengthwise direction a tubular passage which communicates with the tubular passage hole 32. The tubular passage is a container attachment hole 33 to which an integral-type BI 1m is attached. The thickness t of the insertion portion 31 from the outer peripheral surface to the tubular passage hole 32 is set to such a value that heat conduction from the endoscope flexible portion to the tubular passage provided in the endoscope can be reproduced.

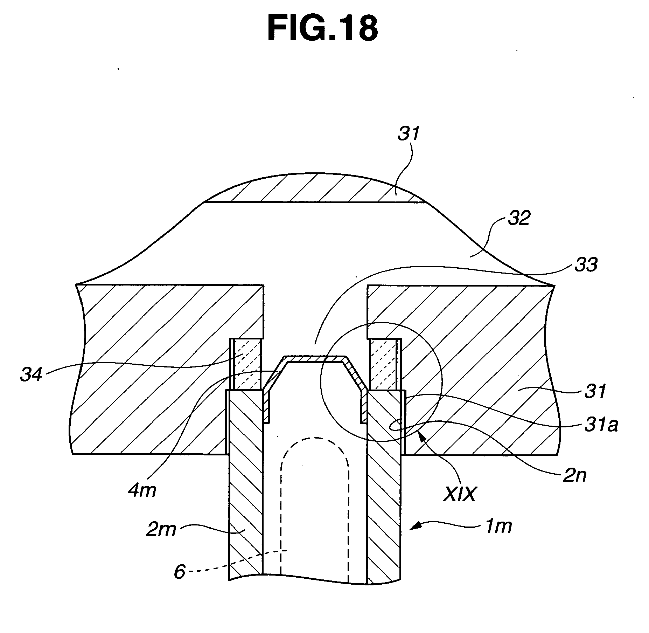

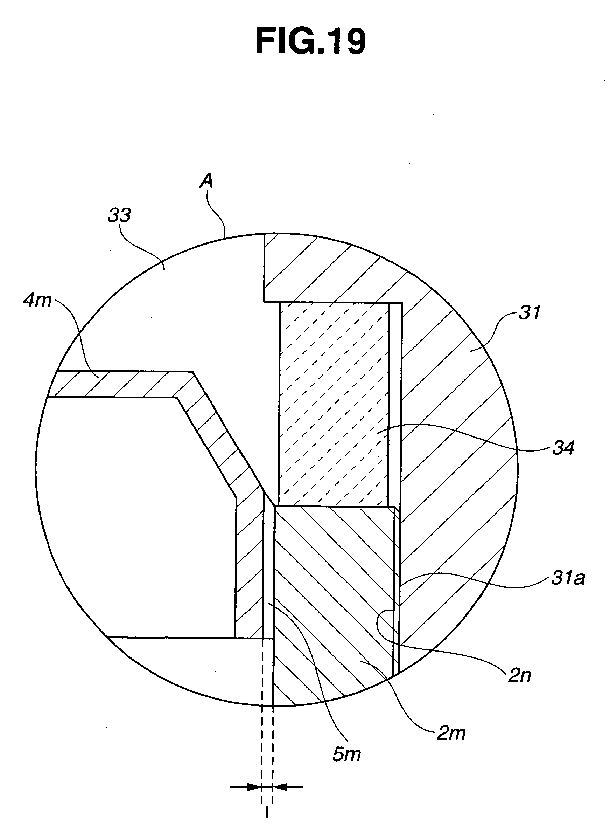

The container attachment hole 33 provided in the side surface of the insertion portion main body 31 has a recessed portion. As shown in Figs. 18 and 19, the recessed portion has in its recessed inner peripheral surface a female threaded portion 31a to be engaged with a male threaded portion 2n, and a packing member 34 which the integral-type BI 1m contacts is disposed in the recessed portion. The container attachment hole 33 is provided generally at the center of the insertion portion main body 31.

Meanwhile, integral-type BI 1 m is constituted by a cylindrical container 2m, a culture solution capsule 6 housed in the container 2m, index germ paper 7 not shown in the figure, and a cap 4m attached on an opening of the container 2m. The cap 4m has a plurality of sterilizing gas penetration ports 5m at the boundary on the container 2m corresponding to its outer peripheral surface. An opening width 1 of the sterilizing gas penetration ports 5m is set such that sterilizing gas can penetrate into the container 2m while the index germs and the culture solution in the container 2m do not flow out.

The container 2m is formed with a comparatively large wall thickness. The cap 4m seals in the culture solution capsule 6 and the index germ paper 7 housed on the container 2m.

The integral-type BI 1m has the male threaded portion 2n on the external surface of the container 2m on the opening side. The distal end surface of the container 2m airtightly contacts the annular packing member 34 provided in the container attachment hole 33. The wall thickness of the container 2m is set to such a value that a state of heat conduction similar to the relationship between the exterior and the tubular passage of the endoscope flexible portion is obtained.

That is, the endoscope sterilization evaluation device 30 according to the present embodiment is mounted such that the male threaded portion 2n provided on the container 2m on which the cap 4m of the integral-type BI 1m is attached is screw-engaged with the female threaded portion 31a of the container attachment hole 33 of the insertion portion main body 31 in which the packing member 34 is fitted in advance. The integral-type BI 1m is thereby attached to the container attachment hole 33 in an airtight manner to provide communication between the tubular passage hole 32 of the insertion portion main body 31 and the sterilizing gas penetration ports 5m of the integral-type BI 1m.

The endoscope sterilization evaluation device 30 according to the present embodiment has a reduced number of component parts constituting the entire device and can be simply formed in comparison with the endoscope sterilization evaluation device 11 according to the above-described first embodiment. After undergoing sterilization processing under the same conditions as the endoscope while being placed in the sterilization unit, the integral-type BI 1m can be easily taken out of the container attachment hole 33 of the insertion portion main body 31. As described above, the culture solution capsule 6 is broken to cause the culture solution to leak out and the index germ paper 7 is immersed in the culture solution and cultivated at a predetermined temperature for a predetermined time period, followed by evaluation of the sterilization effect.

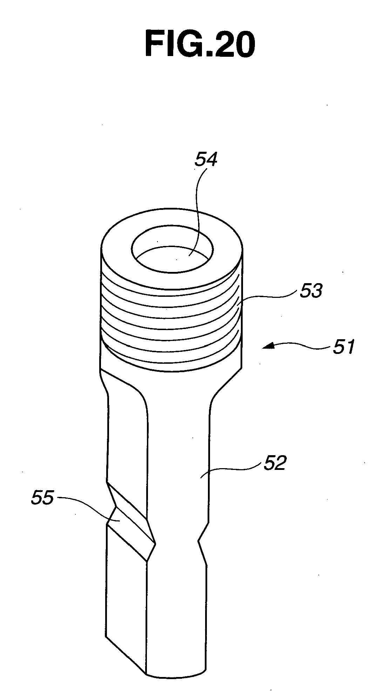

An endoscope sterilization evaluation device according to a fourth embodiment of the present invention will be described with reference to Figs. 20 to 21.

The endoscope sterilization evaluation device according to the fourth embodiment uses, in place of the above-described integral-type BI 1, a biological indicator (hereinafter referred to simply as "BI") 51 in which only index germ paper 7' provided by attaching index germs to a medium such as paper or cloth or impregnating the medium with the index germs is housed in a container. In the BI 51, it is necessary to determine the sterilization effect by immersing in the culture solution the index germ paper 7' taken out of the container after sterilization processing with sterilizing gas and performing cultivation. Accordingly, the present embodiment is the same as the first embodiment except for the configuration in which the BI 51 is connected to the branch tube 17a of the T-branch connection tube 17. Only points of difference from the first embodiment will be described.

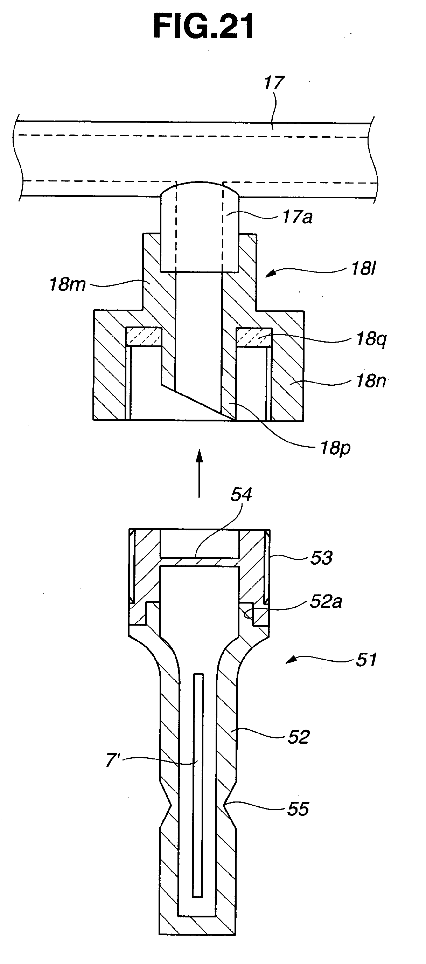

As shown in Figs. 20 and 21, the BI 51 is constituted by a cylindrical head portion 53 and a rectangular container portion 52. The head portion 53 has a male thread on the outer peripheral surface on the opening side. A thin film portion 54 is provided as a partition wall in an upper surface central portion of the head portion 53. The container portion 52 is formed integrally with the head portion 53 by bonding or welding. Breaking grooves 55 are formed in the container portion 52 perpendicularly to the lengthwise direction of the container portion 52 generally at a center in the lengthwise direction. The portion in the container portion 52 corresponding to the breaking grooves 55 is made thinner than other portions due to the breaking grooves 55. The thickness of the portion in the container portion 52 in which the breaking grooves 55 are provided is set to such a value that the container portion 52 is broken along the breaking grooves 55 to be separated into two when a predetermined external force is applied thereto. Screw-engaging portion 52a are formed in portions of the container portion 52 and the head portion 53 to be jointed to each other and the container portion 52 and the head portion 53 are airtightly screw-engaged and bonded to each other.

That is, after housing the index germ paper 7' in the container portion 52, the head portion 53 and the container portion 52 are airtightly screw-engaged and bonded to each other. The index germ paper 7' is thereby housed in the container portion 52 in a sealed-in state. If in this state the container portion 52 is broken along the breaking grooves 55 of the container portion 52, the index germ paper 7' housed in the container portion 52 can be taken out of the broken portions.

On the other hand, a mount portion main body 181 provided on the branch tube 17a of the T-branch connection tube 17 communicating with the tubular passage bodies 13 has a substantially convex shape as viewed in section in Fig. 21 and airtightly bonded and fixed to the branch tube 17a. The mount portion main body 181 has a branch tube attachment portion 18m, a piercing portion 18p in projecting form having a distal end sharply formed, and a BI mount portion 18n.

The branch tube 17a is bonded and fixed to the branch tube attachment portion 18m. The branch tube attachment portion 18m and the piercing portion 18p have tubular passages communicating with the branch tube 17a. The BI mount portion 18n has a recess in which the head portion 53 of the BI 51 is placed. The recess has in its inner peripheral surface a female threaded portion with which the male thread provided on the head portion 53 is screw-engaged. A packing member 18q formed of an elastic member is placed on the bottom surface of the recess.

The external size of the piercing portion 18p is smaller in diameter than the thin film portion and has predetermined rigidity. The piercing portion 18p is thus prevented from being crushed when caused to pierce through the thin film portion 54 of the BI 51.

That is, the BI 51 is mounted to the BI mount portion 18n of the mount portion main body 181. When the BI 51 is mounted, the head portion 53 of the BI 51 is attached to the BI mount portion 18n by screw-engagement. At that time, the piercing portion 18p of the mount portion main body 181 is caused to penetrate into the container 52 by breaking the thin film portion 54 of the BI 51. Communication is thereby provided between the T-branch connection tube 17 and the interior of the BI 51 in which the index germ paper 7' is housed. At this time, the upper surface of the head portion 53 of the BI 51 and the packing member 18q in the mount portion main body 181 are brought into close contact with each other. The interior of the BI 51 is thereby enabled to communicate only with the T-branch connection tube 17.

The BI 51 is thus mounted to the endoscope sterilization evaluation device having the tubular passage bodies 13 diverging from the T-branch connection tube 17 and undergoes predetermined sterilization processing by a sterilization unit. Thereafter, the container portion 52 of the BI 51 is broken at the breaking portion 55, and the index germ paper 7' housed in the container portion 52 is taken out and moved into a container containing a culture solution. That is, the index germ paper 7' housed in the container portion 52 can be moved to the container containing the culture solution without being touched.

Thus, after sterilization processing has been performed on the BI 51, i.e., the biological indicator in which only the index germ paper 7' is housed, even an operator not skilled in the cultivation operation on the BI 51 processed by sterilization processing can perform the operation to immerse the index germ paper 7' in the culture solution without touching the index germ paper 7', and determine the effect of sterilization processing.

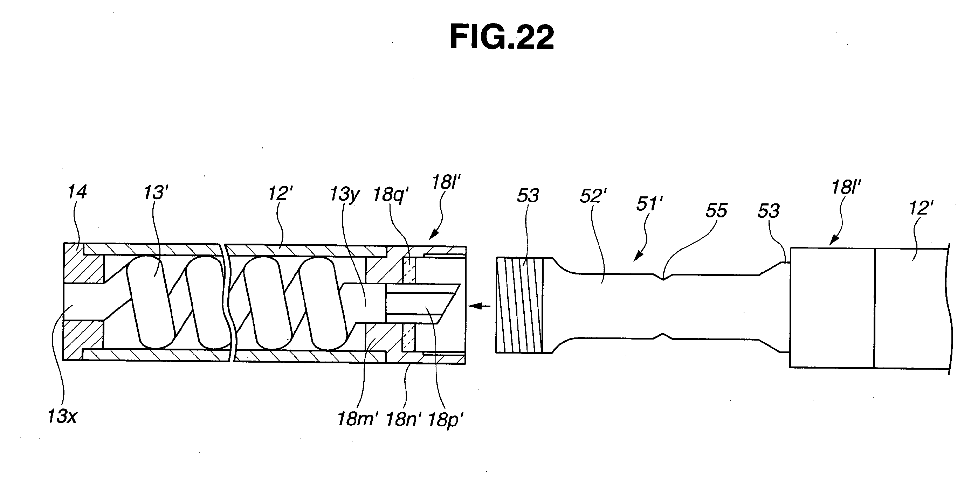

An endoscope sterilization evaluation device according to a fifth embodiment of the present invention will be described with reference to Fig. 22.

The endoscope sterilization evaluation device or the like according to the above-described first embodiment has an increased length. Therefore, if the space in a chamber of a sterilization unit is extremely small, the space may be occupied by the device so that it may be difficult to accommodate the endoscope sterilization evaluation device in the chamber together with the endoscope to be subjected to sterilization processing. In such a case, only the endoscope sterilization evaluation device is first accommodated in the sterilization unit, sterilization processing is performed thereon, and the conditions of drive of the sterilization unit and the sterilization processing conditions are checked. Thereafter, sterilization processing is performed on the endoscope device. A considerably complicated process is required to do so.

The present embodiment is an endoscope sterilization evaluation device for improving such a complicated process. When the endoscope sterilization evaluation device is accommodated in a sterilization unit, the space occupied by the device is minimized to enable sterilization evaluation thereon under the same conditions as an endoscope also subjected to sterilization processing.

In the endoscope sterilization evaluation device according to the fifth embodiment, a tubular passage body 13' simulating the tubular passage in the endoscope and placed by being inserted in a flexible tube member 12' simulating the exterior of the above-described flexible portion of the endoscope is spirally formed. The length of the spiral tubular passage body 13' corresponds to the length of the portion of the endoscope which is under such tubular passage conditions as to be hard to sterilize, and the corresponding length of the tubular passage body 13' is spirally formed. One end 13x of the spiral tubular passage body 13' is configured to communicate with the outside. That is, one end 13x of the spiral tubular passage body 13' is airtightly fixed to an end constituent member 14 airtightly fixed to one end portion of the flexible tube member 12'.

The other end 13y of the spiral tubular passage body 13' is a communication opening to which a mount portion main body 181' of the same configuration as that of the mount portion main body 181 described with reference to Fig. 21 is airtightly connected. The other end of the flexible tube member 12' is airtightly fixed to the mount portion main body 181.

A pair of flexible tube members 12 incorporating spiral tubular passage bodies 13' thus configured are prepared and each mount portions 181' and a BI 51' are placed. In the BI 51' in the present embodiment, the head portion 53 of the BI 51 described above with reference to Figs. 20 and 21 is provided on each of opposite ends of a container portion 52'. That is, when the mount portions 181', 181' of the flexible tube members 12', 12' are screw-mounted to the two head portions 53, 53 of the BI 51', the thin film portions 54 of the BI 51 are broken by piercing portions 18p', 18p' of the mount portions 181', 181' to provide communication between the interior of the container portion 52 and the tubular passage bodies 13'.

Thus, the tubular passage body 13' having a length corresponding to the length of an internal portion of the endoscope which is under such tubular passage conditions as to be hard to sterilize is spirally formed and provided in the flexible tube member 12', thereby reducing the size of the entire configuration of the endoscope sterilization evaluation device. Consequently, the space occupied by the endoscope sterilization evaluation device in a chamber of a sterilization unit is reduced to enable the endoscope sterilization evaluation device to be subjected to sterilization processing together with the endoscope. As a result, the reliability of determination of the effect of sterilization processing is improved.

It is needless to say to place the spiral tubular passage body 13' provided in the flexible tube member 12' in consideration of heat conduction between the exterior of the endoscope insertion portion and the tubular passage in the endoscope insertion portion, and it is apparent that this arrangement can also be applied to any of the flexible tube members 12 and the tubular passage bodies 13 of the first to fourth embodiments described above.

The present invention is not limited to the above-described embodiments. Various modifications can be made within the scope not departing from the gist of the invention.

The present application is made as the basis for claim of priority of Japanese Patent Application No. 2005-158164 filed in Japan on the date of May, 30, 2005. The contents of the above disclosure are cited in the specification, the claims and the accompanying drawings in the present application.

## Claims

1. An endoscope sterilization evaluation device comprising:
an elongated hollow body having a hollow portion opened at opposite ends, the elongated hollow body simulating an endoscope having a tubular passage;
an exterior member having a first housing portion in which a biological indicator for performing sterilization evaluation on the hollow body is housed, and a second housing portion in which a culture solution for cultivating germs attached to the biological indicator is housed, the exterior member also having at a predetermined position a communication passage for communication between the first housing portion and the outside; and
mount means for holding the communication passage in a state of communicating with the hollow portion, and mounting the exterior member to the hollow body.

2. An endoscope sterilization evaluation device comprising:
an elongated hollow body having a hollow portion opened at opposite ends, the elongated hollow body simulating an endoscope having a tubular passage;
an exterior member having a housing portion in which a biological indicator for performing sterilization evaluation on the hollow body is housed, and in which a culture solution housing member containing in a sealing manner a culture solution for cultivating germs attached to the biological indicator is housed, and also having at a predetermined position a communication passage for communication between the housing portion and the outside; and
mount means for holding the communication passage in a state of communicating with the hollow portion, and mounting the exterior member to the hollow body.

3. The endoscope sterilization evaluation device according to claim 1, further comprising a branch passage branching from the hollow portion, wherein the mount means mounts the exterior member to the hollow body by setting the communication passage in a state of communicating with the branch passage.

4. The endoscope sterilization evaluation device according to claim 1 or 2, wherein the hollow body has an elongated hollow flexible tube member simulating the exterior of an insertion portion of the endoscope, and a tubular passage body simulating a tubular passage provided in the endoscope and opened at opposite ends, and
wherein the tubular passage body is inserted and placed in a hollow in the flexible tube member.

5. The endoscope sterilization evaluation device according to claim 4, further comprising tube holding ribs for holding the tubular passage body in a state of being spaced apart by a predetermined distance from the inner periphery of the flexible tube member.

6. The endoscope sterilization evaluation device according to claim 4, wherein the flexible tube member is formed into a bellows-like shape.

7. The endoscope sterilization evaluation device according to claim 4, wherein the tubular passage body is spirally formed.

8. The endoscope sterilization evaluation device according to claim 1, wherein the exterior member has a partition wall portion which isolates the first housing portion and the second housing portion from each other, and the partition wall portion has a thin portion for breaking the partition wall portion.

9. The endoscope sterilization evaluation device according to claim 2, wherein the exterior member is formed of a flexible resin material, and the culture solution housing member is breakable with deformation of the exterior member.

10. The endoscope sterilization evaluation device according to claim 1, wherein the hollow body has a predetermined thickness such as to have heat conduction substantially equivalent to heat conduction to the tubular passage in the endoscope.
